# FASCICULE DE BREVET EUROPEEN

(11) **EP 0 676 217 B1**
(45) Date de publication et mention de la délivrance du brevet: **28.08.2002**
(21) Numéro de dépôt: 95400756.3
(22) Date de dépôt: 05.04.1995
(51) Int. Cl.: A61N 1/368

(54) **Simulateur cardiaque auriculaire double du type triple chambre programmable en mode de repli**
Doppeltvorhof-Dreikammerherzschrittmacher mit Fallback-Modus
Double atrial pacemaker of triple-chamber type programmable in fallback mode

(30) Priorité: 05.04.1994 FR 9403987
(43) Date de publication de la demande: 11.10.1995
(73) Titulaire: ELA MEDICAL (Société anonyme), F-92541 Montrouge (FR)
(72) Inventeur: Limousin, Marcel, F-91120 Montrouge (FR); Remy, Martine, F-78350 Jouy en Josas (FR)
(74) Mandataire: Dupuis-Latour, Dominique

(56) Documents cités:
- WO-A-92/09331
- WO-A-92/14511
- US-A- 5 107 850

## Description

L'invention concerne les stimulateurs cardiaques du type "triple chambre", c'est-à-dire ceux permettant une stimulation du ventricule droit et de chacune des deux oreillettes, droite et gauche. Elle concerne plus particulièrement les stimulateurs cardiaques de ce type possédant un mode de fonctionnement en "repli" (*fallback*) propre à désynchroniser la stimulation ventriculaire lorsque le rythme auriculaire est trop rapide, puis resynchroniser progressivement celle-ci lorsque ce rythme auriculaire redevient moins rapide.

En effet, si le patient développe une crise de tachycardie auriculaire (également appelée "tachycardie supra-ventriculaire" ou TSV), il est nécessaire de limiter la synchronisation à une plage donnée afin d'éviter une stimulation trop rapide du ventricule au-delà d'une fréquence maximale supportable par l'individu, appelée "fréquence maximale de stimulation ventriculaire". Pour ce faire, le stimulateur pourvu d'un mode de fonctionnement en "repli" essaye de garder une certaine synchronisation au-delà de la fréquence maximale et, si la fréquence auriculaire continue à dépasser la fréquence maximale de synchronisation, il déclenche un mode de stimulation asynchrone, tendant vers une fréquence ventriculaire plus basse. Dès que la fréquence auriculaire repasse au-dessous de la limite maximale de synchronisation, une phase de resynchronisation progressive est entamée. Le FR-A-2 544 989 décrit un tel mode de fonctionnement, appliqué au cas d'un stimulateur de type "double chambre".

La présente invention vise le cas particulier où un tel mode de "repli" est incorporé à un stimulateur cardiaque du type "triple chambre".

Les stimulateurs de ce type sont ceux qui comportent, outre une sonde ventriculaire (généralement bipolaire), deux sondes auriculaires implantées sur chacune des deux oreillettes et reliées à une borne correspondante, unique, du stimulateur par l'intermédiaire d'un connecteur en Y. La sonde auriculaire est ainsi une sonde double mais, à la différence des électrodes d'une sonde "bipolaire" classique, où les deux extrémités distale et proximale ne sont éloignées que de quelques millimètres, les deux extrémités des sondes sont relativement éloignées, d'une valeur typique de l'ordre de 5 cm. Certains de ces appareils sont en outre pourvus d'une commutation entre un mode de détection et de stimulation bipolaire et un mode monopolaire. Une telle commutation peut être réalisée par programmation extérieure, et elle peut agir sur l'étage auriculaire comme sur l'étage ventriculaire d'un stimulateur double chambre.

Les stimulateurs cardiaques triple chambre sont utilisés de façon relativement satisfaisante depuis quelques années dans les indications de patients présentant un dysfonctionnement sinusal du type "bloc inter-auriculaire" conduisant à une propagation déficiente (insuffisante ou trop longue) de l'oreillette droite à l'oreillette gauche. Ainsi, si l'on ne stimulait que l'une des oreillettes (situation classique des stimulateurs "double chambre"), l'oreillette gauche, non stimulée, recevrait l'onde de dépolarisation provenant de l'oreillette droite, stimulée, avec un délai excessivement long, quelquefois plus long que le délai auriculo-ventriculaire. Un tel phénomène peut conduire à une contraction des ventricules survenant avant vidange de l'oreillette gauche, donc avant fermeture de la valvule mitrale, produisant ainsi un contre-flux sanguin du ventricule vers l'oreillette et une diminution de l'efficacité hémodynamique. De plus, la désynchronisation électrique des deux oreillettes favorise l'apparition de tachyarythmies. En outre, le délai inter-auriculaire augmentant avec l'effort, l'accroissement de l'activité physiologique du patient favorise le risque d'apparition d'un tel syndrome. Les stimulateurs "triple chambre", en stimulant l'une et l'autre oreillette de façon simultanée, évitent l'apparition ou la persistance de ce type de phénomènes.

Toutefois, les études cliniques ont révélé, pour certains patients, un fonctionnement parfois défectueux du mode de repli, avec une période de désynchronisation se prolongeant même après le retour du rythme auriculaire en-deçà de la limite maximale de synchronisation. Ces dysfonctionnements, dont l'origine n'avait jusqu'à présent pas pu être décelée, pouvaient même aller jusqu'à imposer une désactivation du mode de repli, et donc obliger le praticien à choisir entre un fonctionnement du type "triple chambre sans repli" ou bien du type "double chambre avec repli", donc sans pouvoir combiner, avec toutes les garanties de sécurité nécessaires, fonctionnement triple chambre et mode de repli.

L'un des buts de l'invention est de proposer un stimulateur cardiaque "triple chambre" permettant d'activer un mode de repli en éliminant tout risque de dysfonctionnement, donc de bénéficier à la fois des avantages de la stimulation triple chambre et de la possibilité d'un fonctionnement en repli en cas de rythme auriculaire excessif, notamment en cas de crise de tachycardie auriculaire.

L'invention est essentiellement basée sur la découverte de la cause probable des dysfonctionnements associés aux stimulateurs triple chambre opérant en mode de repli qui, comme on l'expliquera plus en détail par la suite, sont très vraisemblablement la conséquence d'une confusion intervenant, dans certaines situations, sur l'origine des signaux auriculaires recueillis par les circuits de détection auriculaire du stimulateur. En effet, le stimulateur reçoit sur une seule et même entrée deux signaux différents et successifs correspondant, le premier, au front de dépolarisation issu de l'oreillette droite et, le second, à ce même front de dépolarisation capté par la sonde placée sur l'oreillette gauche, donc avec un retard correspondant au temps de propagation inter-auriculaire. Dans certaines situations, notamment si le temps de propagation inter-auriculaire est important, ces deux signaux peuvent se présenter sous la forme de doublets, donc de deux signaux distincts. Le circuit de détection auriculaire du stimulateur peut, dans certains cas, interpréter ― à tort ― ce doublet comme une succession de deux ondes de dépolarisation auriculaire successives et, compte tenu de leur succession rapide, croire à un rythme auriculaire très rapide et maintenir en conséquence le stimulateur en mode de repli, alors même que le rythme auriculaire réel serait descendu en-deçà de la limite maximale devant permettre d'entamer la resynchronisation progressive.

L'objet de l'invention est de procéder à une discrimination appropriée permettant d'éviter de confondre un doublet provenant d'un seul et même front d'onde de dépolarisation auriculaire avec des signaux auriculaires consécutifs, et d'éviter ainsi la persistance du mode de repli, dysfonctionnement spécifique de la stimulation triple chambre dont l'origine était jusqu'à présent inconnue.

L'invention s'applique, comme on vient de le dire, à un stimulateur cardiaque auriculaire double du type triple chambre, c'est-à-dire comprenant les caractéristiques visées au préambule de la revendication 1.

Selon l'invention, il comprend les moyens énoncés par la partie caractérisante de la revendication 1.

Les sous-revendications 2 et 3 visent des mises en oeuvre avantageuses.

D'autres caractéristiques et avantages de l'invention apparaîtront à la lecture de la description ci-dessous, faite en référence aux dessins annexés.

La figure 1 est une vue schématique des différents organes d'un stimulateur triple chambre et de l'implantation de ses sondes sur le myocarde.

La figure 2 illustre la succession dans le temps des divers fronts d'onde de dépolarisation recueillis par le circuit de détection auriculaire du stimulateur, permettant d'expliciter le principe de l'invention.

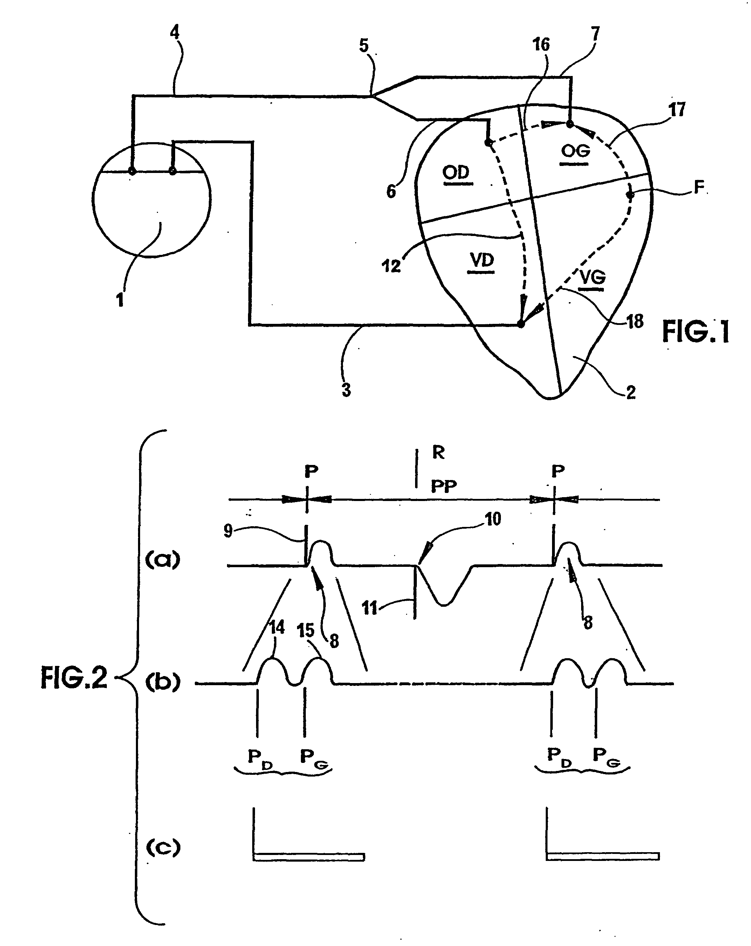

Sur la figure 1, la référence 1 désigne le stimulateur cardiaque, qui est d'un type classique DDD ou DDT, c'est-à-dire un stimulateur double bipolaire (détection des signaux auriculaires et ventriculaires et stimulation auriculaire et ventriculaire), fonctionnant en mode déclenché, c'est-à-dire produisant une stimulation dès le recueil d'un signal sur la sonde correspondante ou, de façon imposée, en l'absence de recueil d'un signal après écoulement d'un laps de temps donnés.

Le stimulateur 1 est relié au myocarde 2 par une configuration du type triple chambre, c'est-à-dire avec une sonde ventriculaire 3 reliée au ventricule droit VD et une sonde auriculaire 4 reliée à chacune des deux oreillettes, droite OD et gauche OG par l'intermédiaire d'une bifurcation en Y, référencée 5, et de deux branches de sondes respectives 6 et 7. Du fait de ce montage en Y, le circuit de détection/stimulation auriculaire du stimulateur relié à la sonde 4 (en entrée pour la détection et en sortie pour la stimulation) détecte tout signal recueilli par l'une quelconque des sondes 6 ou 7 indifféremment et, inversement, stimule simultanément et de façon identique les deux oreillettes.

Le signal recueilli par ce circuit auriculaire se présente généralement de la manière illustrée figure 2a, et comprend une succession d'ondes auriculaires (ondes P) telles que celle référencée 8, l'apparition d'une telle onde déclenchant immédiatement une stimulation auriculaire, correspondant au pic 9. Le circuit recueille ensuite l'onde ventriculaire 10 associée au pic 11 en cas de stimulation produite par la sonde 3. Le décalage temporel entre onde P et onde R correspond au délai atrio-ventriculaire (schématisé par la flèche 12 sur la figure 1). Bien entendu, en cas de bloc atrio-ventriculaire ou anomalie du même type, le circuit ventriculaire du stimulateur déclenche automatiquement la stimulation si aucune onde ventriculaire n'est détectée au bout d'un laps de temps prédéterminé.

Dans le cas particulier de la stimulation triple chambre, le front de dépolarisation auriculaire 8 peut se présenter, en l'absence de stimulation, de la manière illustrée à plus grande échelle figure 2b, c'est-à-dire sous la forme d'un doublet 14,15, l'onde 14 correspondant à l'onde P de l'oreillette droite et l'onde 15, à l'onde P de l'oreillette gauche. L'espacement entre les deux ondes 14 et 15 dépend du délai inter-auriculaire (schématisé en 16 sur la figure 1), qui est variable selon les sujets et qui, chez un même sujet, augmente avec l'effort.

Par ailleurs, le stimulateur comporte un mode de fonctionnement en "repli", susceptible de provoquer une désynchronisation de la stimulation ventriculaire, par exemple par allongement du délai auriculo-ventriculaire jusqu'à diminution du rythme de cette stimulation jusqu'à une fréquence de base supportable par l'individu (fréquence de base qui peut être prédéterminée par le stimulateur, ou bien asservie à un signal issu d'un capteur physiologique). La resynchronisation intervient ultérieurement, et de façon progressive, lorsque la fréquence auriculaire repasse au-dessous de la limite maximale de synchronisation. Ce mode de fonctionnement, dans ses diverses variantes qui ont pu être proposées, est en lui même bien connu, par exemple d'après le FR-A-2 544 989 précité, et ne sera pas décrit plus en détail.

Dans le cas particulier d'un stimulateur triple chambre, le signal recueilli par le circuit de détection auriculaire peut se présenter, comme on l'a indiqué plus haut, sous la forme d'un doublet tel qu'illustré figure 2b lorsque le couplage (c'est-à-dire le paramètre inverse du délai séparant les deux signaux du doublet) entre les deux composantes du doublet est faible (délai typique de l'ordre de 200 ms), ce qui signifie qu'une même onde de dépolarisation auriculaire peut être détectée deux fois. Ce faible couplage a pour conséquence que le stimulateur risque d'interpréter les deux détections successives 14,15 non pas comme le doublet associé à une même onde auriculaire, mais comme deux ondes auriculaires successives, et croire ainsi à une fréquence auriculaire élevée (un intervalle de 200 ms correspondant en effet à un rythme de 300 min⁻¹) et prolonger ainsi le mode de repli.

Pour remédier à cette situation, selon une première forme de mise en oeuvre de l'invention, à chaque réception d'un signal de détection auriculaire, on déclenche une période réfractaire suffisamment longue (par exemple d'une durée de 200 ms) pour masquer le temps de propagation inter-auriculaire. Cette période réfractaire, illustrée sur la figure 2c, est une période spécifique, qui peut être mise en oeuvre de manière en elle-même connue par programmation appropriée du microprocesseur du stimulateur ou bien par des circuits dédiés à compteur et bascules.

De la sorte, si le front d'onde reçu qui a déclenché la période réfractaire est le front de dépolarisation auriculaire directement capté sur l'oreillette droite (c'est-à-dire une onde telle que celle illustrée en 14 sur la figure 2b), la période réfractaire spécifique sera suffisamment longue pour masquer l'onde 15 correspondant à la même dépolarisation mais captée sur l'oreillette gauche avec un retard correspondant au délai de propagation inter-auriculaire ; on évite ainsi toute fausse interprétation du doublet 14,15 et donc tout maintien à tort de la désynchronisation du mode de repli.

La période réfractaire spécifique sera suffisamment courte pour ne pas masquer la réception de la prochaine onde de dépolarisation auriculaire ; l'intervalle entre deux signaux auriculaires captés par le stimulateur correspondra alors en toute certitude au rythme auriculaire réel et le programme du stimulateur pourra ainsi déterminer ― à bon escient et de manière en elle-même classique ― s'il y a lieu de maintenir le mode de repli ou, au contraire, de resynchroniser progressivement la stimulation ventriculaire sur le rythme auriculaire.

En variante, selon une seconde forme de réalisation de l'invention, on masque la réception du doublet en commutant l'étage de détection auriculaire du mode bipolaire en mode unipolaire quand l'appareil est en mode de repli. De cette façon, l'une des électrodes auriculaires est reliée au boîtier du stimulateur et l'autre à une oreillette (par exemple la droite). Dans cette configuration, il ne peut plus y avoir réception d'un doublet, et on évite ainsi toute confusion entre les signaux.

## Revendications

1. Un stimulateur cardiaque auriculaire double (1) du type triple chambre, comportant une sonde auriculaire droite(6) et une sonde auriculaire gauche (7) reliées ensemble à un seul et même circuit de détection/stimulation auriculaire, ainsi qu'une sonde ventriculaire (3) reliée à un circuit de détection/stimulation ventriculaire, ce stimulateur comportant en outre un mode de désynchronisation de la stimulation ventriculaire lorsque le rythme auriculaire est trop rapide puis de resynchronisation progressive en cas de retour du rythme auriculaire à un rythme moins rapide, stimulateur **caractérisé en ce qu'**il comprend :
a) des moyens aptes à recevoir en entrée du circuit auriculaire une succession de signaux ;
b) des moyens aptes, à chaque détection d'un signal, à masquer un délai de propagation inter-auriculaire ;
c) des moyens aptes à mesurer l'intervalle de temps ainsi masqué séparant deux signaux successifs ;
d) des moyens aptes à comparer cet intervalle de temps masqué à une valeur de consigne prédéterminée ; et
e) des moyens aptes à déclencher le mode de resynchronisation progressive lorsque l'intervalle de temps masqué ainsi mesuré redevient supérieur à la valeur de consigne.

2. Le stimulateur de la revendication 1, dans lequel les moyens de masquage du délai de propagation inter-auriculaire sont mis en oeuvre par déclenchement d'une période réfractaire spécifique.

3. Le stimulateur de la revendication 1, dans lequel les moyens de masquage du délai de propagation inter-auriculaire sont mis en oeuvre par commutation de la détection des signaux auriculaires d'un mode bipolaire à un mode monopolaire, et dans lequel il est en outre prévu :
f) des moyens de commutation inverse de la détection des signaux auriculaires d'un mode monopolaire à un mode bipolaire après déclenchement du mode de resynchronisation progressive.

## Patentansprüche

1. Doppelvorkammer-Herzschrittmacher (1) des Drei-Kammer-Typs, mit einer rechten Vorhofkammersonde (6) und einer linken Vorhofkammersonde (7), die gemeinsam verbunden sind mit ein- und derselben Vorhofkammer-Detektions/Stimulations-Schaltung, sowie einer Herzkammersonde (3), die verbunden ist mit einer Herzkammer-Detektions/Stimulations-Schaltung, wobei der Schrittmacher des Weiteren einen Modus zum Entsynchronisieren der Herzkammerstimulation aufweist, wenn der Vorhofkammer-Rhythmus zu schnell ist, danach zum progressiven Wiedersynchronisieren im Fall der Rückkehr des Vorhofkammer-Rhythmus zu einem weniger schnellen Rhythmus,
wobei der Schrittmacher **dadurch gekennzeichnet ist, dass** er aufweist:
a) Mittel, geeignet zum Empfangen am Eingang der Vorhofkammer-Schaltung, einer Signalfolge;
b) Mittel, geeignet, um bei jeder Detektion eines Signals eine Laufzeitverzögerung zwischen den Vorhofkammern zu maskieren;
c) Mittel, geeignet zum Messen des so maskierten Zeitintervalls, welches zwei sukzessive Signale separiert;
d) Mittel, geeignet zum Vergleichen des maskierten Zeitintervalls mit einem vorbestimmten Einstellwert; und
e) Mittel, geeignet zum Auskoppeln des Modus der progressiven Wiedersynchronisation, wenn das so gemessene maskierte Zeitintervall wieder größer als der Einstellwert wird.

2. Schrittmacher gemäß Anspruch 1, bei welchem die Mittel zum Maskieren der Laufzeitverzögerung zwischen den Vorhofkammern in Betrieb gesetzt werden durch Auslösen einer spezifischen Widerstandsperiode.

3. Schrittmacher gemäß Anspruch 1, bei welchem die Mittel zum Maskieren der Laufzeitverzögerung zwischen den Vorhofkammern in Betrieb gesetzt werden durch Umschalten der Detektion der Vorhofkammersignale von einem bipolaren Modus in einen monopolaren Modus und bei welchem des Weiteren vorgesehen ist:
f) Mittel zum inversen Umschalten der Detektion der Vorhofkammersignale von einem monopolaren Modus in einen bipolaren Modus nach Auslösen des progressiven Wiedersynchronisationsmodus.

## Claims

1. A double auricular heart stimulator (1) of the triple-chamber type, comprising a right auricular probe (6) and a left auricular probe (7) connected together at a single auricular detection/stimulation circuit, and a ventricular probe (3) connected to a ventricular detection/stimulation circuit, the stimulator further having a mode in which ventricular stimulation is desynchronized whenever the auricular rate is too fast and then progressively resynchronized when the auricular rate returns to a slower rate,
the stimulator being **characterized in that** it comprises:
a) means suitable for receiving a succession of signals from the auricular circuit;
b) means suitable, each time a signal is detected, for masking an inter-auricular propagation delay;
c) means suitable for measuring the time interval as masked in this way between two successive signals;
d) means suitable for comparing this masked time interval with a predetermined reference value; and
e) means suitable for triggering progressive resynchronization mode when the masked time interval as measured becomes greater than the reference value.

2. The stimulator of claim 1, in which the means for masking the inter-auricular propagation delay are implemented by triggering a specific refractory period.

3. The stimulator claim 1, in which the means for masking the inter-auricular propagation time are implemented by switching auricular signal detection from a bipolar mode to a monopolar mode, and in which there are also provided:
f) means for inverse switching of auricular signal detection from a monopolar mode to a bipolar mode after the progressive resynchronization mode has been triggered.
